# EUROPEAN PATENT SPECIFICATION

(11) **EP 2 685 246 B1**
(45) Date of publication and mention of the grant of the patent: **20.05.2015**
(21) Application number: 12755431.9
(22) Date of filing: 08.03.2012
(51) Int. Cl.: G01N 23/05, F16C 19/16, F16C 19/52, F16C 41/00, G01M 13/04, G01N 23/09

(54) **LUBRICANT DISTRIBUTION ACQUISITION DEVICE AND LUBRICANT DISTRIBUTION ACQUISITION METHOD**
VORRICHTUNG ZUR ERFASSUNG EINER SCHMIERMITTELVERTEILUNG UND VERFAHREN ZUR ERFASSUNG EINER SCHMIERMITTELVERTEILUNG
DISPOSITIF ET PROCÉDÉ D'ACQUISITION DE DISTRIBUTION DE LUBRIFIANT

(30) Priority: 10.03.2011 JP 2011053438
(43) Date of publication of application: 15.01.2014
(73) Proprietor: IHI Corporation, Tokyo 135-8710 (JP)
(72) Inventor: MOCHIKI Koh-ichi, Tokyo 158-8557 (JP); NOSE Hiroyuki, Tokyo 135-8710 (JP); ITO Akira, Tokyo 135-8710 (JP); TAKANO Takehisa, Tokyo 135-8710 (JP)
(74) Representative: Lamb, Martin John Carstairs
(86) International application number: PCT/JP2012/055957
(87) International publication number: WO 2012/121329

(56) References cited:
- WO-A1-2007/105366
- JP-A- 2000 292 373
- JP-A- 2000 292 373
- JP-A- 2000 292 374
- JP-A- 2005 069 288
- JP-A- 2007 248 325
- JP-A- 2010 054 500
- JP-A- 2010 286 069
- JOHN T.LINDSAY ET AL.: 'X-ray vision on steroids' MACHINE DESIGN vol. 70, no. 6, April 1998, pages 49 - 51, XP008171668

## Description

### [Field of the Invention]

The present invention relates to a lubricant distribution acquisition device and a lubricant distribution acquisition method.

Priority is claimed on Japanese Patent Application No. 2011-53438, filed March 10, 2011,

### [Background Art]

For example, in Patent document 1, an invention is disclosed that uses neutron radiography to examine whether or not a lubricant is present inside a hydrodynamic bearing.

By using an invention of the type that is disclosed in Patent document 1, without dismantling the bearing it has become possible to perform an examination to determine whether or not a lubricant is present which hitherto has required the bearing to be dismantled.

### [Related Art Documents]

### [Patent Documents]

[Patent document 1] Japanese Patent Application, First Publication No. 2000-292373

### [Disclosure of the Invention]

### [Problems to be Solved by the Invention]

However, because the invention disclosed in Patent document 1 only examines whether or not a lubricant is present, and does not detect the behavior of the lubricant, there is no synchronization between the rotation of the bearing and the timing of the imaging. Because of this, if there are any irregularities in the rotation speed of the bearing or if there are changes due to elapsed time, then the pitch of the rotation angle will not be uniform in each set of imaging data, and the ability to compare and contrast different sets of imaging data is impaired.

The present invention was conceived in view of the above-described drawback, and it is an object thereof to provide a lubricant distribution acquisition device and a lubricant distribution acquisition method that make the pitch of the rotation angle uniform in each set of imaging data, and thereby make it possible to accurately ascertain the behavior of a lubricant inside a bearing.

### [Means for Solving the Problems]

The applicants for the present invention conducted research into the relationship between the behavior of a lubricant inside a bearing and the lifespan of the bearing. As a result, they discovered that individual differences existed between the lifespans of different bearings even when the environment and the like in which they were used were the same. When these bearings having different lifespans were dismantled and examined, it was found that there were considerable differences in the state of the lubricant present inside them. In a roller bearing, in particular, it was found that the behavior of the lubricant inside the bearing had a huge effect on the lifespan.

This suggests that the lifespan of a bearing depends on the behavior of the lubricant inside it. Namely, if the behavior of the lubricant inside a bearing can be ascertained, then there is a possibility that the lifespan of the bearing may be able to be improved.

Based on these research results, a first aspect of the present invention employs a constitution in which a lubricant distribution acquisition device is provided with: an electromagnetic wave converting means that receives neutron beams that have been transmitted through a bearing, and then converts these neutron beams into electromagnetic waves; an imaging processing means that, by receiving the electromagnetic waves emitted from the electromagnetic wave converting device and using these electromagnetic waves to form images, acquires lubricant distribution data that shows the distribution of a lubricant inside the bearing; an encoder that outputs a rotation angle signal that shows a rotation angle of the bearing; and a control means that controls timings of the imaging performed by the imaging processing means based on the rotation angle signal.

A second aspect of the present invention is the above-described first aspect of the present invention wherein a constitution is employed in which there is provided an electromagnetic wave amplifying means that amplifies the electromagnetic waves emitted from the electromagnetic wave converting means before they reach the imaging processing means, and the control means causes the electromagnetic wave amplifying means to amplify the electromagnetic waves so as to match the imaging timings.

A third aspect of the present invention is the above-described second aspect of the present invention wherein a constitution is employed in which the start timing of the exposure period of the imaging processing means is set to be delayed beyond the start timing of the electromagnetic wave amplification of the electromagnetic wave amplifying device by a period that is longer than an after image period of the electromagnetic wave amplifying means.

A fourth aspect of the present invention is any one of the above-described first through third aspects of the present invention wherein a constitution is employed in which the bearing is a roller bearing in which at least one rolling body is formed from a material whose neutron beam absorption rate is different from that of the other rolling bodies, and the control means causes the imaging processing means to acquire a plurality of sets of imaging data by causing it to form images at previously determined set angles of rotation, and also causes the imaging processing means to calculate amounts of slippage of the rolling bodies using the plurality of sets of imaging data.

A fifth aspect of the present invention is a lubricant distribution acquisition method wherein a constitution is employed in which neutron beams that have been transmitted through a bearing are converted into electromagnetic waves, and, by using the received electromagnetic waves to form images based on rotation angle signals that are output from an encoder and show a rotation angle of the bearing, lubrication distribution data that shows the distribution of a lubricant inside the bearing is acquired.

### [Effects of the Invention]

According to the present invention, imaging is performed based on a rotation angle signal which is a signal showing the rotation angle of a bearing and which is output from an encoder.

Because of this, even if there are any irregularities in the rotation speed of the bearing or if there are changes due to elapsed time, it is still possible to perform imaging that is always accurately matched to the rotation angle of the bearing.

Accordingly, it is possible to make the pitch of the rotation angle uniform in each set of imaging data, and to thereby accurately ascertain the behavior of the lubricant inside the bearing.

### [Brief Description of the Drawings]

[FIG. 1A] FIG. 1A shows the schematic structure of a lubricant distribution acquisition device according to an embodiment of the present invention, and is a typical view of a portion of the mechanism thereof.
[FIG. 1B] FIG. 1B shows the schematic structure of the lubricant distribution acquisition device according to an embodiment of the present invention, and is a block diagram showing a portion of the functions thereof.
[FIG. 2] FIG. 2 is a perspective view of a cutaway model showing the schematic structure of a bearing that is installed in the lubricant distribution acquisition device according to an embodiment of the present invention.
[FIG. 3] FIG. 3 is a timing chart showing timings of image acquisitions in the lubricant distribution acquisition device according to an embodiment of the present invention.
[FIG. 4] FIG. 4 is a typical view showing an example of a change in a bearing that is used in the lubricant distribution acquisition device according to an embodiment of the present invention.
[FIG. 5] FIG. 5 is a photograph showing an imaging result from the inside of a bearing according to the present invention.

### [Embodiments for Carrying out the Invention]

An embodiment of the lubricant distribution acquisition device and lubricant distribution acquisition method of the present invention will now be described with reference made to the drawings. Note that in the following drawings, the scale of each component has been suitably altered in order to make each component a recognizable size.

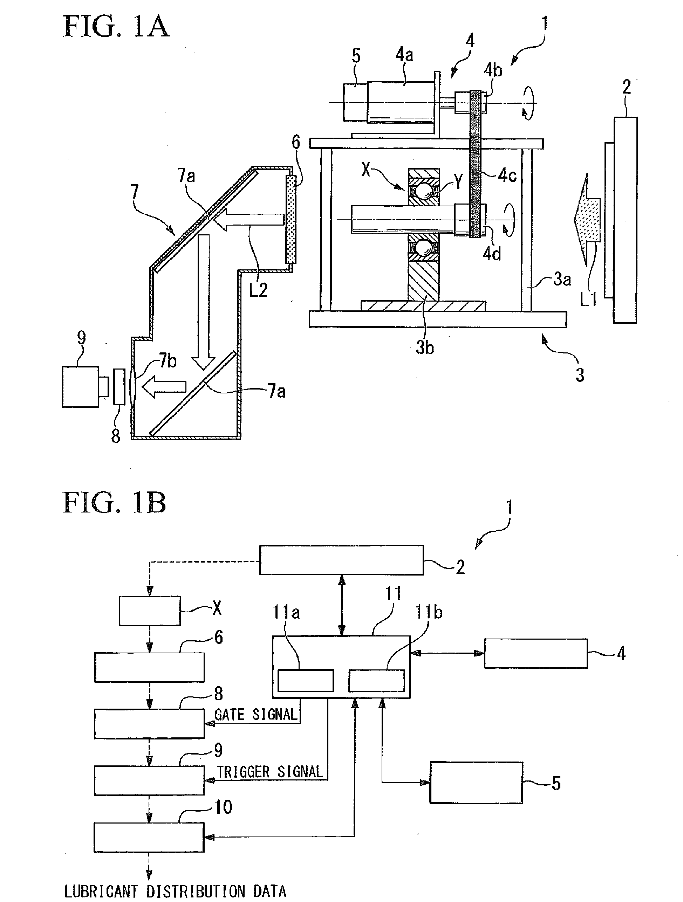

FIGS. 1A and 1B are views showing in typical form the schematic structure of a lubricant distribution acquisition device 1 of the present embodiment. FIG. 1A is a typical view showing a portion of the mechanism of the lubricant distribution acquisition device 1, while FIG. 1B is a block diagram showing a portion of the functions of the lubricant distribution acquisition device 1.

The lubricant distribution acquisition device 1 of the present embodiment ascertains the behavior of a lubricant Y (for example, grease) during the rotation of a bearing X, which is a ball bearing, by acquiring the distribution of the lubricant Y inside the bearing X.

In addition, as is shown in FIGS. 1A and 1B, the lubricant distribution acquisition device 1 of the present embodiment is provided with a neutron beam irradiation device 2, a bearing support mechanism 3, a rotation drive device 4 (i.e., a rotation drive means), a rotary encoder (i.e., an encoder) 5, a scintillator 6 (i.e., an electromagnetic wave converting means), a light guide mechanism 7, a light amplifier 8 (i.e., an electromagnetic wave amplifying means), an imaging device 9, a signal processing section 10, and a control unit 11 (i.e., a control means).

The neutron beam irradiation device 2 guides a neutron beam L1 emitted from a neutron source such as, for example, an atomic reactor so as to irradiate them onto the bearing X from an axial direction.

Note that if it is possible to irradiate neutron beams emitted from the neutron source onto the bearing X from an axial direction without having to guide the neutron beams, then it is also possible to omit the neutron beam irradiation device 2.

Moreover, in the lubricant distribution acquisition device 1 of the present embodiment, it is also possible to provide a separate neutron source that generates neutron beams by irradiating ions of hydrogen or helium or the like that have been generated by an ion generator, for example, onto a target.

The bearing support mechanism 3 is used to support the bearing X, and is provided with a case body 3a and with a housing 3b.

The case body 3a is a frame body or box-shaped component that contains inside it the housing 3b and the bearing X that is fixed to the housing 3b. In the present embodiment, as is shown in FIG. 1A, the case body 3a also functions as a support base for the rotation drive device 4.

The housing 3b is used to cover and support the outer wheel of the bearing X, and supports the bearing X such that the bearing X can be removably connected thereto. In addition, in the present embodiment, as is shown in FIG. 1A, the housing 3b supports the bearing X such that the main axis of the bearing X faces towards the neutron beam irradiation device 2 side.

Note that it is preferable for the case body 3a and the housing 3b to be shaped such that they avoid the transmission area of the neutron beam L1, however, if they are formed from an aluminum material or the like that has an extremely low neutron beam L1 absorption rate, then the case body 3a and the housing 3b may be shaped such that they span across the transmission area of the neutron beam L1.

The rotation drive device 4 is used to drive the bearing X to rotate and, as is shown in FIG. 1A, is provided with a motor 4a (i.e., a motive power unit) that generates motive power for driving the bearing X to rotate, a pulley 4b that is used to transmit the motive power generated by the motor 4a to the bearing X by means of a belt, a belt 4c (i.e., a belt-shaped component), and a driveshaft portion 4d.

More specifically, the pulley 4b is joined by a coupling or the like to a shaft portion of the motor 4a. The driveshaft portion 4d is a rod-shaped component that is elongated in the axial direction of the bearing X. The driveshaft portion 4d is fixed to the inner ring of the bearing X, and is placed horizontally so as to penetrate the center of the bearing X. The belt 4c is formed by an endless belt, and is entrained between the pulley 4b and the driveshaft portion 4d.

The rotary encoder 5 outputs rotation angle signals that show the angle of rotation of the bearing X.

This rotary encoder 5 is connected to a rotation shaft of the motor 4a that rotates in synchronization with the bearing X, and outputs a pulse signal that matches the angle of rotation (i.e., the rotation speed) when the rotation shaft of the motor 4a is driven to rotate. A pulse output (i.e., an incremental) type of rotary encoder, for example, can be used as the rotary encoder 5.

The scintillator 6 is used to receive the neutron beam L1 that is transmitted through the bearing X and to then emit light L2, and converts the neutron beam L1 into visible light.

For example, LiF/ZnS (Ag), BN/ZnS (Ag), Gd₂O₃/ZnS (Ag), Gd₂O₃S (Tb) can be used for the scintillator 6.

The light guide mechanism 7 guides the light L2 emitted from the scintillator 6 to the imaging device 9 via the light amplifier 8.

As is shown in FIG. 1A, the light guide mechanism 7 is provided with a mirror 7a that reflects and guides the light L2, and with a lens 7b that condenses the light L2.

The light amplifier 8 is used to raise the intensity of the light that enters into it via the light guide mechanism 7, and to then output this light. For example, an image intensifier can be used for the light amplifier 8.

In the present embodiment, under the control of the control unit 11, the light amplifier 8 only amplifies the intensity of the light L2 during a period that is commanded by the control unit 11. More specifically, a gate signal that shows a light amplification period is input from the control unit 11 into the light amplifier 8, and the light amplifier 8 amplifies the light L2 based on this gate signal.

Note that in the present embodiment, because the light amplifier 8 only amplifies the intensity of the light L2 during a period that is commanded by the control unit 11, it is not constantly amplifying the light L2, and idle periods are also generated. Because these idle periods are generated, the light amplifier 8 is prevented from becoming scorched, so the durability thereof can be improved. As a consequence, in the light amplifier 8 of the present embodiment, during the period when the light L2 is being amplified, the output thereof is raised.

By raising the output of the light amplifier 8 in this manner, vivid imaging data can be obtained even if the exposure time in the imaging device 9 is curtailed. Moreover, by curtailing the exposure time, imaging data having only minimal blurring can be obtained.

The imaging device 9 is used to receive the light L2 that was emitted from the scintillator 6 and that arrives via the light guide mechanism 7 and the light amplifier 8, and then forms an image using this light. The imaging device 9 outputs the result of this imaging as imaging data.

Note that although a CCD camera, an SIT tube camera, or a high-speed camera or the like can be used for the imaging device 9, because the movement of the lubricant Y inside the bearing X that is rotating at, for example, approximately 6000 rpm is extremely fast, it is preferable for a high-speed camera that is capable of obtaining images at an extremely high frame rate of approximately 2000 fps to be used.

In the present embodiment, under the control of the control unit 11, the imaging device 9 performs imaging at timings that are commanded by the control unit 11. More specifically, a trigger signal that shows an imaging timing is input from the control unit 11 into the imaging device 9, and the imaging device 9 performs the imaging based on this trigger signal.

Note that the response of the light amplifier 8 to the input of the aforementioned gate signal is characterized by being delayed by a fixed period of time. Namely, the light amplifier 8 has a fixed after image period. As a consequence, in the present embodiment, the start timing of the exposure period of the imaging device 9 is set to be delayed for longer than the after image period of the light amplifier 8.

In this manner, by setting the start timing of the exposure period of the imaging device 9 such that it is delayed for longer than the after image period of the light amplifier 8, the imaging process can be prevented from starting before the light L2 is amplified by the imaging device 9.

Note also that the end timing of the exposure period may also be set shorter than the period between the end timing of the light amplification period of the light amplifier 8 and the completion of the after image period.

The signal processing section 10 processes imaging data input from the imaging device 9, and outputs it as requested lubricant distribution data.

The lubricant distribution data referred to here is data that includes information pertaining to the distribution of a lubricant in a radial direction centered on the main axis, and information pertaining to the thickness distribution of the lubricant in an axial direction. The signal processing section 10 of the present embodiment, for example, calculates lubricant distribution data from the brightness information in the imaging data, and performs processing to associate this lubricant distribution data with the detection results from the rotary encoder 5.

Note that because the information pertaining to the distribution of a lubricant in a radial direction centered on the main axis, and information pertaining to the thickness distribution of the lubricant in an axial direction are contained in the actual imaging data itself that was obtained by the imaging device 9, it is also possible for requested lubricant distribution data to be in the form of imaging data. In this case, the signal processing section 10 outputs the imaging data input from the imaging device 9 as lubricant distribution data without modifying it in any way.

Note that in the present embodiment, an imaging processing means of the present invention is formed by both the imaging device 9 and the signal processing section 10.

The control unit 11 is used to control the overall operations of the lubricant distribution acquisition device 1 of the present embodiment and, as is shown in FIG. 1 B, the control unit 11 is electrically connected to the neutron beam irradiation device 2, the rotation drive device 4, the rotary encoder 5, the light amplifier 8, the imaging device 9, and the signal processing section 10.

In addition, the control unit 11 of the present embodiment controls the imaging timings of the imaging device 9 based on rotation angle signals that are input from the rotary encoder 5, and causes the light amplifier 8 to amplify the light L2 so as to match the imaging timings.

More specifically, as is shown in FIG. 1B, the control unit 11 is provided with a frequency divider 11a and a delayer 11b. The control unit 11 creates a trigger signal by dividing the frequency of the rotation angle signal input from the rotary encoder 5 using the frequency divider 11a, and creates a gate signal (i.e., a signal showing the timings for the light amplification by the light amplifier 8) having a temporal width that shows the operating period of the light amplifier with the trigger signal taken as the start point thereof. This gate signal is input into the light amplifier 8.

Moreover, as is described above, in the present embodiment, the start timing of the exposure time of the imaging device 9 is set to a later time than the start timing of the light amplification by considering the after image period of the light amplifier 8. Consequently, the control unit 11 creates an exposure command signal (i.e., a signal showing the timings for the imaging by the imaging device 9) having a temporal width that shows the exposure time using as the start point thereof a point when the trigger signal that was obtained when the frequency divider 11a divided the frequency of the rotation angle signal has been delayed by a fixed time. This exposure command signal is input into the imaging device 9.

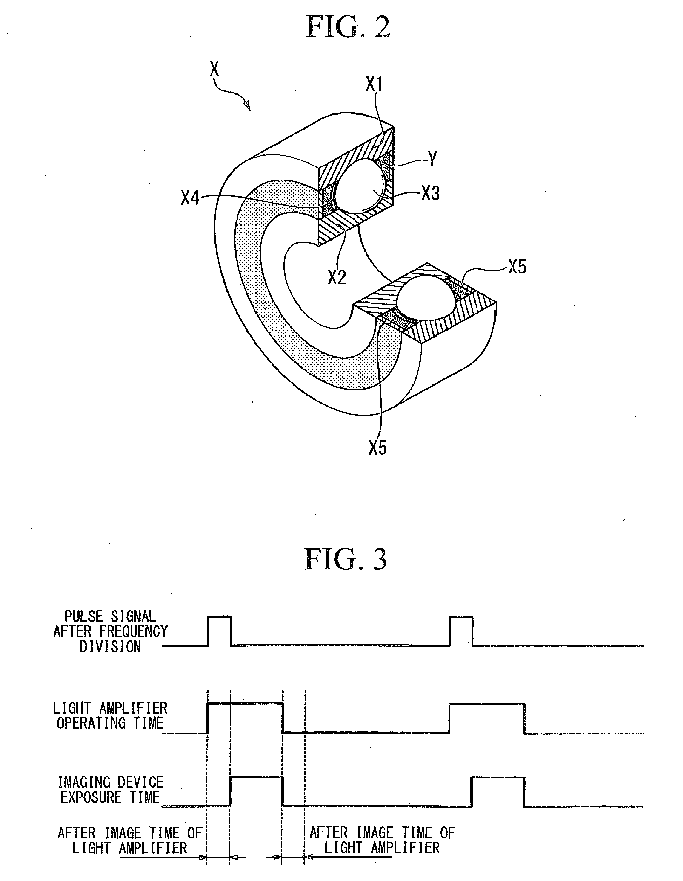

As a result, as is shown in FIG. 3, the light amplifier 8 operates while matching the frequency of the trigger signal after this has undergone frequency division. Note that it is also possible to employ a structure in which the operating period of the light amplifier 8 is stored in advance in the light amplifier 8, and a trigger signal is input directly into the light amplifier 8 from the control unit 11. It is also possible to employ a structure in which the exposure period of the imaging device 9 is stored in advance in the imaging device 9, and a signal that causes the trigger signal to be delayed for a fixed length of time is supplied to the imaging device 9 from the control unit 11. The exposure time of the imaging device 9 is within a range that considers the operating time and the after image period of the light amplifier 8.

Note that in the present embodiment it is a prerequisite that the frequency of the pulse signal that forms the rotation angle signal from the rotary encoder 5 is higher than the frequency of the gate signal and the trigger signal, and for this reason a structure in which the control unit 11 is provided with the frequency divider 11a is employed. However, if the pulse signal output from the rotary encoder 5 is lower than the frequency of the gate signal and trigger signal, then the control unit 11 is provided with a multiplier instead of the frequency divider 11a.

The bearing X is a ball bearing (i.e., a roller bearing) that contains a lubricant inside it and, in the present embodiment, is formed as a radial bearing.

FIG. 2 is a perspective view of a cutaway model showing the schematic structure of the bearing X. As is shown in this drawing, the bearing X is provided with a toroidal outer ring X1 and a toroidal inner ring X2 that are positioned facing each other in a radial direction, a plurality of balls X3 that are located between the outer ring X1 and the inner ring X2, a holder X4 that is used to maintain equidistant intervals between adjacent balls X3, and seals X5 that seal off the spaces where the balls X3 are housed.

Note that in order to raise the visibility of the lubricant Y in the imaging data and to thereby acquire a more accurate distribution, it is desirable that component elements of the bearing X do not appear in the imaging data. Because of this, it is preferable for these component elements of the bearing X (i.e., the outer ring X1, the inner ring X2, the balls X3, the holder X4, and the seals X5) to be formed from an aluminum material that has a low absorption rate of the neutron beam L1.

Next, operations (i.e., a lubricant distribution acquisition method) of the lubricant distribution acquisition device 1 of the present embodiment which is constructed in the manner described above will be described. Note that the main agent of the operations of the lubricant distribution acquisition device 1 of the present embodiment that are described below is the control unit 11.

Firstly, the control unit 11 causes the bearing X to be rotated by the rotation drive device 4. As a result of this, the inner ring X2 of the bearing X is driven to rotate, and the balls X3 that are sandwiched between the inner ring X2 and the outer ring X1 revolve around the main axis at the same time as they are rotated around their own axis. As a consequence, the lubricant Y moves through the interior of the bearing X in conjunction with the movement of the balls X3.

When the bearing X is rotated in this manner, a pulse signal that is formed by a rotation angle signal is input from the rotary encoder 5 into the control unit 11.

The control unit 11 creates a gate signal and a trigger signal from the pulse signal, and inputs the gate signal into the light amplifier 8 and the trigger signal into the imaging device 9.

As a result, the imaging device 9 always performs imaging in synchronization with the rotation angle of the bearing X, and the light amplifier 8 amplifies the light L2 so as to match the timings when the imaging is performed by the imaging device 9.

Next, the neutron beam L1 from the neutron beam irradiation device 2 is guided to the bearing X side. As a result of this, as is shown in FIG. 1A, the neutron beam L1 enters into the bearing X from the axial direction of the bearing X, and the neutron beam L1 that is transmitted through the bearing X then enters into the scintillator 6.

When the neutron beam L1 enters into the scintillator 6, the scintillator 6 emits light the L2 that has the same intensity distribution as the intensity distribution of the neutron beam L1. Namely, the scintillator 6 converts the neutron beam L1 into the light L2 and then emits this light L2.

The light L2 emitted from the scintillator 6 is guided by the light guide mechanism 7 and amplified by the light amplifier 8, and then enters into the imaging device 9.

The control unit 11 then causes the imaging device 9 to create an image. As a result of this, imaging data is acquired by the imaging device 9.

Next, the control unit 11 causes the signal processing section 10 to process the imaging data, and to also calculate lubricant distribution data that includes information pertaining to the distribution of the lubricant in a radial direction centered on the main axis, and information pertaining to the thickness distribution of the lubricant in the axial direction.

The control unit 11 also performs processing to associate the calculated lubricant distribution data with the detection results from the rotation detector 5. As a result of this, the lubricant distribution data is output in association with the rotation angle of the bearing X.

Here, the lubricant Y is formed from an organic material so that it has a higher rate of neutron beam absorption than does the bearing X. Because of this, the neutron beam L1 that has been transmitted through the bearing X is greatly attenuated in areas where the lubricant Y is present. In contrast, the intensity distribution of neutron beam L1 is proportional to the intensity distribution of the light L2 into which the neutron beam L1 has been converted.

Accordingly, by irradiating the neutron beam L1 onto the bearing X from the axial direction, and then acquiring images of the light L2 into which the neutron beam L1 that is transmitted through the bearing X has been converted, it is possible to acquire from the brightness distribution of the image data the distribution of the lubricant Y in a radial direction centered on the main axis.

Moreover, the amount of attenuation of the neutron beam L1 is proportional to the thickness of the lubricant Y in areas through which the neutron beam L1 is transmitted. Namely, the greater the thickness of the lubricant Y in these transmission areas, the greater the amount of attenuation of the neutron beam L1, and the intensity of the neutron beam L after being transmitted through these areas is reduced. In contrast, the intensity distribution of the neutron beam L1 is proportional to the intensity distribution of the light L2 into which the neutron beam L1 is converted. Accordingly, by irradiating the neutron beam L1 onto the bearing X from the axial direction, and then acquiring images of the light L2 into which the neutron beam L1 that is transmitted through the bearing X has been converted, it is possible to acquire from the brightness distribution of the image data the thickness distribution of the lubricant in the axial direction.

In addition, in the lubricant distribution acquisition device 1 and the lubricant distribution acquisition method of the present embodiment, by changing the neutron beam L1 that has been irradiated onto the bearing X from the axial direction and has been transmitted through the bearing X into the light L2, and then forming images from the received light L2, lubricant distribution data that shows the distribution of the lubricant Y inside the bearing X is acquired.

Because of this, according to the lubricant distribution acquisition device 1 and the lubricant distribution acquisition method of the present embodiment, it is possible to acquire lubricant distribution data that includes the distribution of the lubricant Y in a radial direction centered on the main axis and also includes the thickness distribution of the lubricant Y in an axial direction without having to dismantle the bearing X, and it thereby becomes possible to ascertain in detail the behavior of the lubricant Y inside the bearing X.

According to the lubricant distribution acquisition device 1 and the lubricant distribution acquisition method of the present embodiment, imaging is performed based on a rotation angle signal, which is a signal that shows the rotation angle of the bearing X, that is output from the rotary encoder 5.

Because of this, even if there are any irregularities in the rotation speed of the bearing X or if there are changes due to elapsed time, it is still possible to perform imaging that is always accurately matched to the rotation angle of the bearing.

Accordingly, it is possible to make the pitch of the rotation angle uniform in each set of imaging data, and to thereby accurately ascertain the behavior of the lubricant inside the bearing X.

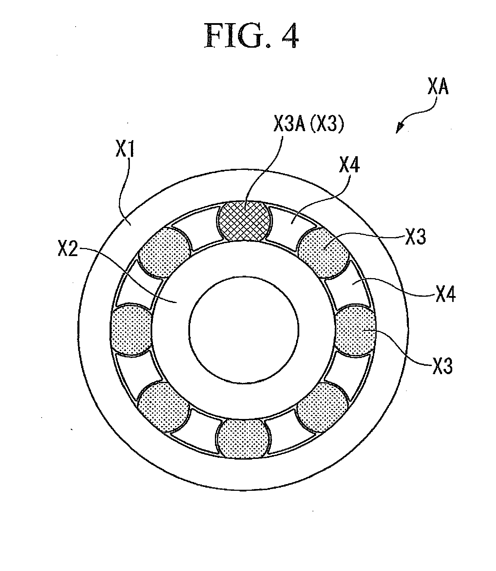

Note that in the lubricant distribution acquisition device 1 of the present embodiment, as is shown in FIG. 4, it is also possible to install a bearing XA in which just one ball X3A (i.e., a moving body) is provided that is formed from a different material (i.e., that has a different neutron beam absorption rate).

By installing this type of bearing XA, it is possible to distinguish the ball X3A from the other balls X3 in the imaging data.

Moreover, when the ball bearing X is rotated, the ball X3 and the ball X3A revolve around the interior of the ball bearing X. The amount of these revolutions can be calculated using the friction force and the like that is acting on the ball X3 and the ball X3A. Therefore, according to the lubricant distribution acquisition device 1 of the present embodiment, because it is possible to make the pitch of the rotation angle uniform in each set of imaging data, it is possible to accurately calculate the amount of revolutions of the ball X3 and the ball X3A from the rotation angle pitches.

As a consequence, by, for example, acquiring a plurality of sets of imaging data, and then causing the signal processing section 10 to calculate the amount of revolutions of the ball X3 and the ball X3A from the rotation angle pitches, and then comparing the positions of the ball X3 and the ball X3A in the actual imaging data, it is possible to calculate the amount of slippage of the ball X3 and the ball X3A.

In this manner, according to the lubricant distribution acquisition device 1 of the present embodiment, by using the bearing XA in which the neutron beam absorption rate of the one ball X3A is different from that of the other balls X3, the amount of slippage of the ball X3 and the ball X3A can be calculated.

Note that it is not essential for only one ball X3A having a different neutron beam absorption rate to be provided and it is also possible for a plurality of these to be provided.

Moreover, it is also not necessary for the neutron beam absorption ratio of the entire ball X3A to be changed. For example, it is also possible to change the neutron beam absorption ratio of the ball X3A by changing the substance of a portion of the ball X3A. The substance that is used to form a portion of the ball X3A may be suitably selected from, for example, iron, aluminum, ceramics and the like.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as limited by the foregoing description and is only limited by the scope of the appended claims.

For example, in the above-described embodiment, a structure in which an incremental encoder is used as the encoder of the present invention is described.

However, the present invention is not limited to this and it is also possible for another encoder such as, for example, an absolute encoder to be used.

For example, in the rotation drive device it is also possible to use a toothed pulley together with a toothed belt. It is also possible for a sprocket (i.e., a wheel portion) and a chain (i.e., a belt-shaped component) to be used.

Moreover, in the above-described embodiment, a structure in which the bearing X is a ball bearing that receives a load in a radial direction is described.

However, it is also possible for the present invention to be used to ascertain the behavior of a lubricant inside other types of bearing such as, for example, roller bearings, sliding bearings, and bearings that receive a load in a thrust direction.

Moreover, in the above-described embodiment, a structure in which the neutron beam L1 is transmitted through a bearing from an axial direction thereof is described.

However, the present invention is not limited to this and it is also possible for a structure in which the neutron beam L1 is transmitted through the bearing from an oblique direction relative to the main axis to be employed.

Moreover, in the above-described embodiment, a structure in which the neutron beam L1 is converted into light L2 using the scintillator 6 is described.

However, the present invention is not limited to this and it is also possible to acquire images by converting the neutron beam L1 into radioactive rays (i.e., electromagnetic waves) such as gamma rays and the like.

Moreover, in the above-described embodiments, a structure in which digital photography is performed by the imaging device 9 is described.

However, the present invention is not limited to this and it is also possible for film photography to be performed by the imaging device.

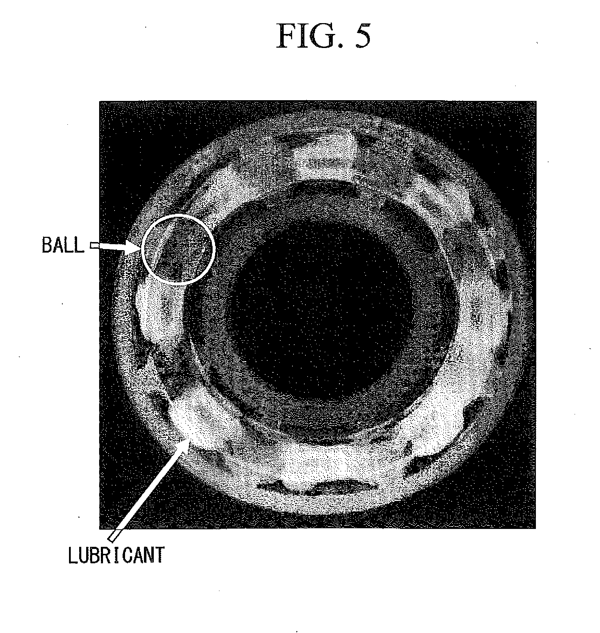

Note that as a result of converting the neutron beam L1 into gamma rays and then performing film photography using the lubricant distribution acquisition device of the present invention, images such as the one shown in FIG. 5 were acquired.

As can be understood from this imaging result, according to the present invention, it is possible to acquire an image of the interior of a bearing.

### [Industrial applicability]

According to the present invention, it is possible to provide a lubricant distribution acquisition device and a lubricant distribution acquisition method that make it possible to accurately ascertain the behavior of a lubricant inside a bearing by making the pitch of the rotation angle uniform in each set of imaging data.

### [Description of the Reference Numerals]

1 ... Lubricant distribution acquisition device, 2 ... Neutron beam irradiation device, 4 ... Rotation drive device, 5 ... Rotary encoder (Encoder), 6 ... Scintillator (Electromagnetic wave converting means), 8 ... Light amplifier (Electromagnetic wave amplifying means), 9 ... Imaging device (Imaging means), 10 ... Signal processing section, 11... Control unit, L1 ... Neutron beam, L2 ... Light (Electromagnetic waves), X, XA ... Bearings, Y ... Lubricant

## Claims

1. A lubricant distribution acquisition device (1) comprising:
an electromagnetic wave converting means (6) that receives neutron beams that have been transmitted through a bearing, and then converts these neutron beams into electromagnetic waves;
an imaging processing means (9) that, by receiving the electromagnetic waves emitted from the electromagnetic wave converting device (6) and using these electromagnetic waves to form images, acquires lubricant distribution data that shows the distribution of a lubricant inside the bearing;
an encoder (5) that outputs a rotation angle signal that shows a rotation angle of the bearing; and
a control means (11) that controls timings of the imaging performed by the imaging processing means (9) based on the rotation angle signal.

2. The lubricant distribution acquisition device (1) according to claim 1 wherein there is provided an electromagnetic wave amplifying means (8) that amplifies the electromagnetic waves emitted from the electromagnetic wave converting means (6) before they reach the imaging processing means (9), and
the control means (11) causes the electromagnetic wave amplifying means (8) to amplify the electromagnetic waves so as to match the imaging timings.

3. The lubricant distribution acquisition device (1) according to claim 2 wherein the start timing of the exposure period of the imaging processing means (9) is set to be delayed beyond the start timing of the electromagnetic wave amplification of the electromagnetic wave amplifying device (8) by a period that is longer than an after image period of the electromagnetic wave amplifying means (8).

4. The lubricant distribution acquisition device (11) according to claim 1 wherein the bearing is a roller bearing in which at least one rolling body is formed from a material whose neutron beam absorption rate is different from that of the other rolling bodies, and
the control means (11) causes the imaging processing means (9) to acquire a plurality of sets of imaging data by causing it to form images at previously determined set angles of rotation, and also causes the imaging processing means (9) to calculate amounts of slippage of the rolling bodies using the plurality of sets of imaging data.

5. The lubricant distribution acquisition device (1) according to claim 2 wherein the bearing is a roller bearing in which at least one rolling body is formed from a material whose neutron beam absorption rate is different from that of the other rolling bodies, and
the control means (11) causes the imaging processing means to acquire a plurality of sets of imaging data by causing it to form images at previously determined set angles of rotation, and also causes the imaging processing means (9) to calculate amounts of slippage of the rolling bodies using the plurality of sets of imaging data.

6. The lubricant distribution acquisition device (1) according to claim 3 wherein the bearing is a roller bearing in which at least one rolling body is formed from a material whose neutron beam absorption rate is different from that of the other rolling bodies, and
the control means (11) causes the imaging processing means (9) to acquire a plurality of sets of imaging data by causing it to form images at previously determined set angles of rotation, and also causes the imaging processing means (9) to calculate amounts of slippage of the rolling bodies using the plurality of sets of imaging data.

7. A lubricant distribution acquisition method in which neutron beams that have been transmitted through a bearing are converted into electromagnetic waves, and, by using the received electromagnetic waves to form images based on rotation angle signals that are output from an encoder (5) and show a rotation angle of the bearing, lubrication distribution data that shows the distribution of a lubricant inside the bearing is acquired.

## Patentansprüche

1. Schmiermittelverteilungserfassungsvorrichtung (1), umfassend:
ein Konvertiermittel für elektromagnetische Wellen (6), das Neutronenstrahlen empfängt, die durch ein Lager gesendet worden sind, und diese Neutronenstrahlen dann in elektromagnetische Wellen konvertiert;
ein Bildverarbeitungsmittel (9), das durch Empfangen der vom Konvertiermittel für elektromagnetische Wellen (6) emittierten elektromagnetischen Wellen und Verwenden dieser elektromagnetischen Wellen, um Bilder zu formen, Schmiermittelverteilungsdaten erfasst, welche die Verteilung eines Schmiermittels innerhalb des Lagers zeigen;
einen Codierer (5), der ein Drehwinkelsignal ausgibt, das einen Drehwinkel des Lagers zeigt; und
ein Steuerungsmittel (11), das auf der Grundlage des Drehwinkelsignals die Zeitvorgaben der durch das Bildverarbeitungsmittel (9) durchgeführten Bilderzeugung steuert.

2. Schmiermittelverteilungserfassungsvorrichtung (1) nach Anspruch 1, worin ein Verstärkungsmittel für elektromagnetische Wellen (8) vorgesehen ist, das die vom Konvertiermittel für elektromagnetische Wellen (6) emittierten elektromagnetischen Wellen verstärkt, bevor sie das Bildverarbeitungsmittel (9) erreichen, und
das Steuerungsmittel (11) das Verstärkungsmittel für elektromagnetische Wellen (8) veranlasst, die elektromagnetischen Wellen so zu verstärken, dass sie zu den Bilderzeugungszeitvorgaben passen.

3. Schmiermittelverteilungserfassungsvorrichtung (1) nach Anspruch 2, worin die Anfangszeitvorgabe der Belichtungsperiode des Bildverarbeitungsmittels (9) so eingestellt wird, dass sie über die Anfangszeitvorgabe der Verstärkung elektromagnetischer Wellen des Verstärkungsmittels für elektromagnetische Wellen (8) hinaus verzögert wird, und zwar um eine Periode, die länger als eine Nachbildperiode des Verstärkungsmittels für elektromagnetische Wellen (8) ist.

4. Schmiermittelverteilungserfassungsvorrichtung (1) nach Anspruch 1, worin das Lager ein Wälzlager ist, bei dem mindestens ein Wälzkörper aus einem Material geformt ist, dessen Neutronenstrahlabsorptionsrate sich von jener der anderen Wälzkörper unterscheidet, und
das Steuerungsmittel (11) das Bildverarbeitungsmittel (9) veranlasst, eine Vielzahl von Bilderzeugungsdatensätzen zu erfassen, indem es dieses veranlasst, Bilder bei vorher bestimmten, eingestellten Drehwinkeln zu bilden, und außerdem das Bildverarbeitungsmittel (9) veranlasst, unter Verwendung der Vielzahl von Bilderzeugungsdatensätzen Schlupfbeträge der Wälzkörper zu berechnen.

5. Schmiermittelverteilungserfassungsvorrichtung (1) nach Anspruch 2, worin das Lager ein Wälzlager ist, bei dem mindestens ein Wälzkörper aus einem Material geformt ist, dessen Neutronenstrahlabsorptionsrate sich von jener der anderen Wälzkörper unterscheidet, und
das Steuerungsmittel (11) das Bildverarbeitungsmittel (9) veranlasst, eine Vielzahl von Bilderzeugungsdatensätzen zu erfassen, indem es dieses veranlasst, Bilder bei vorher bestimmten, eingestellten Drehwinkeln zu bilden, und außerdem das Bildverarbeitungsmittel (9) veranlasst, unter Verwendung der Vielzahl von Bilderzeugungsdatensätzen Schlupfbeträge der Wälzkörper zu berechnen.

6. Schmiermittelverteilungserfassungsvorrichtung (1) nach Anspruch 3, worin das Lager ein Wälzlager ist, bei dem mindestens ein Wälzkörper aus einem Material geformt ist, dessen Neutronenstrahlabsorptionsrate sich von jener der anderen Wälzkörper unterscheidet, und
das Steuerungsmittel (11) das Bildverarbeitungsmittel (9) veranlasst, eine Vielzahl von Bilderzeugungsdatensätzen zu erfassen, indem es dieses veranlasst, Bilder bei vorher bestimmten, eingestellten Drehwinkeln zu bilden, und außerdem das Bildverarbeitungsmittel (9) veranlasst, unter Verwendung der Vielzahl von Bilderzeugungsdatensätzen Schlupfbeträge der Wälzkörper zu berechnen.

7. Schmiermittelverteilungserfassungsverfahren, bei dem Neutronenstrahlen, die durch ein Lager gesendet worden sind, in elektromagnetische Wellen konvertiert werden, und durch Verwenden der empfangenen elektromagnetischen Wellen, um auf der Grundlage von Drehwinkelsignalen, die von einem Codierer (5) ausgegeben werden und einen Drehwinkel des Lagers zeigen, Bilder zu formen, Schmiermittelverteilungsdaten erfasst werden, welche die Verteilung eines Schmiermittels innerhalb des Lagers zeigen.

## Revendications

1. Dispositif d'acquisition de répartition de lubrifiant (1) comprenant
un moyen de conversion d'onde électromagnétique (6) qui reçoit des faisceaux de neutrons qui ont été émis à travers un palier, et convertit ensuite ces faisceaux de neutrons en ondes électromagnétiques ;
un moyen de traitement par imagerie (9) qui, en recevant les ondes électromagnétiques émises à partir du dispositif de conversion d'onde électromagnétique (6) et en utilisant ces ondes électromagnétiques pour former des images, acquiert des données de répartition de lubrifiant qui montrent la répartition d'un lubrifiant à l'intérieur du palier ;
un codeur (5) qui produit un signal d'angle de rotation qui montre un angle de rotation du palier ; et
un moyen de commande (11) qui commande des temporisations de l'imagerie mise en oeuvre par le moyen de traitement par imagerie (9) en se basant sur le signal d'angle de rotation.

2. Dispositif d'acquisition de répartition de lubrifiant (1) selon la revendication 1, dans lequel est fourni un moyen d'amplification d'onde électromagnétique (8) qui amplifie les ondes électromagnétiques émises à partir du moyen de conversion d'onde électromagnétique (6) avant qu'elles atteignent le moyen de traitement par imagerie (9), et
le moyen de commande (11) amène le moyen d'amplification d'onde électromagnétique (8) à amplifier les ondes électromagnétiques de manière à correspondre aux temporisations d'imagerie.

3. Dispositif d'acquisition de répartition de lubrifiant (1) selon la revendication 2, dans lequel la temporisation de départ de la période d'exposition du moyen de traitement par imagerie (9) est réglée de manière à être différée après la temporisation de départ de l'amplification d'onde électromagnétique du dispositif d'amplification d'onde électromagnétique (8) à raison d'une période qui est plus longue qu'une période d'après-image du moyen d'amplification d'onde électromagnétique (8).

4. Dispositif d'acquisition de répartition de lubrifiant (1) selon la revendication 1, dans lequel le palier est un palier à rouleaux dans lequel au moins un corps roulant est formé à partir d'un matériau dont la vitesse d'absorption d'un faisceau de neutrons est différente de celle des autres corps roulants, et
le moyen de commande (11) amène le moyen de traitement par imagerie (9) à acquérir une pluralité de séries de données d'imagerie en l'amenant à former des images pour des angles de rotation réglés, préalablement déterminés, et amène également le moyen de traitement par imagerie (9) à calculer des valeurs de glissement des corps roulants en utilisant la pluralité de séries de données d'imagerie.

5. Dispositif d'acquisition de répartition de lubrifiant (1) selon la revendication 2, dans lequel le palier est un palier à rouleaux dans lequel au moins un corps roulant est formé à partir d'un matériau dont la vitesse d'absorption d'un faisceau de neutrons est différente de celle des autres corps roulants, et
le moyen de commande (11) amène le moyen de traitement par imagerie (9) à acquérir une pluralité de séries de données d'imagerie en l'amenant à former des images pour des angles de rotation réglés, préalablement déterminés, et amène également le moyen de traitement par imagerie (9) à calculer des valeurs de dérapage des corps roulants en utilisant la pluralité de séries de données d'imagerie.

6. Dispositif d'acquisition de répartition de lubrifiant (1) selon la revendication 3, dans lequel le palier est un palier à rouleaux dans lequel au moins un corps roulant est formé à partir d'un matériau dont la vitesse d'absorption d'un faisceau de neutrons est différente de celle des autres corps roulants, et
le moyen de commande (11) amène le moyen de traitement par imagerie (9) à acquérir une pluralité de séries de données d'imagerie en l'amenant à former des images pour des angles de rotation réglés, préalablement déterminés, et amène également le moyen de traitement par imagerie (9) à calculer des valeurs de glissement des corps roulants en utilisant la pluralité de séries de données d'imagerie.

7. Procédé d'acquisition de répartition de lubrifiant dans lequel des faisceaux de neutrons qui ont été émis à travers un palier sont convertis en ondes électromagnétiques, et des données de répartition de lubrification qui montrent la répartition d'un lubrifiant à l'intérieur du palier sont acquises grâce à l'utilisation des ondes électromagnétiques reçues pour former des images en se basant sur des signaux d'angle de rotation qui sont produits par un encodeur (5) et qui montrent un angle de rotation du palier.
